(19) Europäisches Patentamt
European Patent Office
Office européen des brevets

(11) EP 3 016 942 B1

(12) **EUROPEAN PATENT SPECIFICATION**

(45) Date of publication and mention of the grant of the patent:
**01.03.2017 Bulletin 2017/09**

(21) Application number: **14735951.7**

(22) Date of filing: **03.07.2014**

(51) Int Cl.:
***C07D 311/22*** *(2006.01)*

(86) International application number:
**PCT/EP2014/064210**

(87) International publication number:
**WO 2015/001031 (08.01.2015 Gazette 2015/01)**

(54) **FORMATION OF CHIRAL 4-CHROMANONES USING CHIRAL PYRROLIDINES**

HERSTELLUNG VON CHIRALEN 4-CHROMANONEN UNTER VERWENDUNG VON CHIRALEN PYRROLIDINEN

FORMATION DE CHIRAUX 4-CHROMANONES UTILISANT DES PYRROLIDINES CHIRAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.07.2013 EP 13175317**

(43) Date of publication of application:
**11.05.2016 Bulletin 2016/19**

(73) Proprietor: **DSM IP Assets B.V.**
**6411 TE Heerlen (NL)**

(72) Inventors:
- **ACKERMANN, Stephan**
**CH-4002 Basel (CH)**
- **LETINOIS, Ulla**
**CH-4002 Basel (CH)**

(74) Representative: **Dux, Roland**
**DSM Nutritional Products Ltd**
**Patent Department**
**Wurmisweg 576**
**4303 Kaiseraugst (CH)**

(56) References cited:
**US-A- 4 415 741**

- **KABBE H J ET AL: "Synthesen und Umsetzungen von 4-Chromanonen", ANGEWANDTE CHEMIE, WILEY - V C H VERLAG GMBH & CO. KGAA, DE, vol. 94, 1 January 1982 (1982-01-01), pages 254-262, XP002418875, ISSN: 0044-8249**
- **PEARCE B C ET AL: "INHIBITORS OF CHOLESTEROL BIOSYNTHESIS. 2. HYPOCHOLESTEROLEMIC AND ANTIOXIDANT ACTIVITIES OF BENZOPYRAN AND TETRAHYDRONAPHTHALENE ANALOGUES OF THE TOCOTRIENOLS.OPYRAN AND TETRAHYDRONAPHTHALENE", JOURNAL OF MEDICINAL CHEMISTRY, AMERICAN CHEMICAL SOCIETY, vol. 37, no. 4, 18 February 1994 (1994-02-18), pages 526-541, XP002015864, ISSN: 0022-2623, DOI: 10.1021/JM00030A012**
- **RIOS ET AL: "Highly enantioselective synthesis of 2H-1-benzothiopyrans by a catalytic domino reaction", TETRAHEDRON LETTERS, PERGAMON, vol. 47, no. 48, 27 November 2006 (2006-11-27), pages 8547-8551, XP005720620, ISSN: 0040-4039, DOI: 10.1016/J.TETLET.2006.09.135**
- **NUNEZ M G ET AL: "Asymmetric organocatalytic synthesis of six-membered oxygenated heterocycles", TETRAHEDRON, ELSEVIER SCIENCE PUBLISHERS, AMSTERDAM, NL, vol. 66, no. 12, 20 March 2010 (2010-03-20), pages 2089-2109, XP026915103, ISSN: 0040-4020, DOI: 10.1016/J.TET.2009.12.045 [retrieved on 2010-01-04]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 016 942 B1

## Description

### Technical Field

The present invention relates to the field of the synthesis of tocopherols and tocotrienols.

### Background of the invention

Chromane compounds represent an important class of chiral natural products and bioactive compounds. An important class of chromane compounds are vitamin E and its esters. Often vitamin E is commercialized in the form of its esters because the latter show an enhanced stability.

On the one hand the typical technical synthesis of vitamin E leads to mixtures of isomers. On the other hand higher bioactivity (biopotency) has been shown to occur in general by tocopherols and tocotrienols having the R-configuration at the chiral centre situated next to the ether atom in the ring of the molecule (indicated by * in the formulas used later on in the present document) (i.e. 2R-configuration), as compared to the corresponding isomers having S-configuration. Particularly active are the isomers of tocopherols having the natural configuration at all chiral centres, for example (R,R,R)-tocopherols, as has been disclosed for example by H. Weiser et al. in J. Nutr. 1996, 126(10), 2539-49. This leads to a strong desire for an efficient process for separating the isomers. Hence, the isomer separation not only of vitamin E, but also of their esters, particularly their acetates, as well as of their precursors is of prime interest.

Separation of all the isomers by chromatographic methods is extremely difficult and costly.

To overcome these inherent problems, it has been tried to offer stereospecific synthesis allowing the preferential formation of the desired isomers only. However, these methods are very expensive, complex and/or exotic as compared to the traditional industrial synthesis leading to isomer mixtures.

Therefore, there exists a large interest in providing stereospecific synthesis routes leading to the desired isomer.

Particular difficult is to achieve specifically the desired chirality at the chiral carbon centre in the 2-position of the chromane ring.

A synthetic pathway for chromanes is via their corresponding chromanones. It is known from Kabbe and Heitzer, Synthesis 1978; (12): 888-889 that α-tocopherol can be synthesized via α-tocotrienol from 4-oxo-α-tocotrienol which is accessible from 2-acetyl-3,5,6-trimethylhydroquinone and farnesyl-acetone in the presence of pyrrolidine. However, this procedure leads to a racemic mixture in view of the configuration at the 2-position of the chromane respectively chromanone ring.

### Summary of the invention

Therefore, the problem to be solved by the present invention is to offer a method for the synthesis of chromanones or chromanes, i.e. of compounds of formula (I) or (V) in a stereospecific matter in view of the 2-position in the chromanone or chromane ring.

Surprisingly, it has been found that a process for the manufacturing according to claim 1 is able to solve this problem.

It has been particularly found that the use of a specific chiral compound leads to the formation of the desired product and a desired stereoselective formation. Particularly, the desired isomer is formed in preference over the non-desired isomer yielding to an enantiomeric ratio being larger than zero, or a ratio of [2R]-stereoisomers to [2S]-stereoisomers being larger than one.

Further aspects of the invention are subject of further independent claims. Particularly preferred embodiments are subject of dependent claims.

### Detailed description of the invention

In a first aspect the present invention relates to a process for the manufacturing of a compound of formula (I)

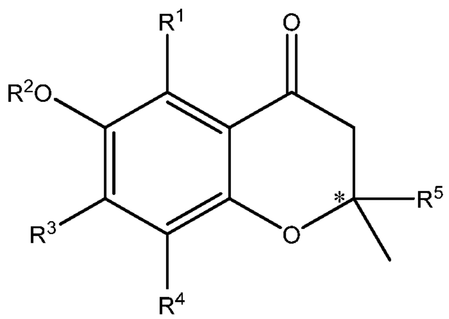

(I)

comprising the step of reacting a composition consisting of

- compound of formula (II-A);
- compound of formula (II-B) or a ketal thereof and
- at least one chiral compound of formula (II-C)

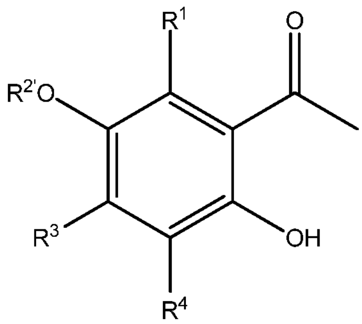

(II-A)

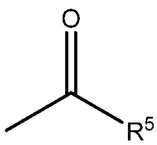

(II-B)

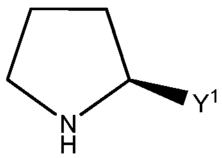

(II-C)

wherein $R^1$, $R^3$ and $R^4$ are independently from each other hydrogen or methyl groups;
$R^2$ and $R^2$ represents hydrogen or a phenol protection group;
$R^5$ represents either a linear or branched completely saturated $C_{6-25}$-alkyl group or a linear or branched $C_{6-25}$-alkyl group comprising at least one carbon-carbon double bond;
$Y^1$ represents either $CH_2Y^2$ or

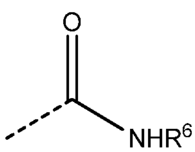

wherein $R^6$ represents a linear or branched $C_{1-12}$-alkyl group which optionally further comprises at least one aromatic group and/or C=O and/or NH and/or $NH_2$ group;

$Y^2$ represents either OH or $OR^7$ or $NHR^7$ or $NHCOOR^7$ or

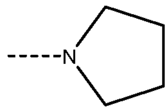

wherein $R^7$ represents either

a linear or branched $C_{1-12}$-alkyl group which optionally further comprises at least one aromatic group and/or C=O and/or NH and/or $NH_2$ group
or
an aryl group or a substituted aryl group or a heteroaryl group or a substituted heteroaryl group;

and the dotted line(s) represents the bond(s) by which the corresponding substituent is bound to the rest of formula (II-C);
and wherein * represents the chiral centre of the chiral isomer of formula (I).

The term "independently from each other" in this document means, in the context of substituents, moieties, or groups, that identically designated substituents, moieties, or groups can occur simultaneously with a different meaning in the same molecule.
A "$C_{x-}$y-alkyl", resp. "$C_{x-y}$-acyl" group, is an alkyl resp. an acyl group comprising x to y carbon atoms, i.e. for example an $C_{1-3}$-alkyl group, is an alkyl group comprising 1 to 3 carbon atoms. The alkyl resp. the acyl group can be linear or branched. For example $-CH(CH_3)-CH_2-CH_3$ is considered as a $C_4$-alkyl group.
A "$C_{x-y}$-alkylene" group is an alkylene group comprising x to y carbon atoms, i.e., for example $C_2$-$C_6$ alkylene group is an alkyl group comprising 2 to 6 carbon atoms. The alkylene group can be linear or branched. For example the group $-CH(CH_3)-CH_2-$ is considered as a $C_3$-alkylene group.
The term "hydrogen" means in the present document H and not $H_2$.
The sign * in formulae of molecules represents in this document a chiral centre in said molecule.
In the present document any single dotted line represents the bond by which a substituent is bound to the rest of a molecule.
The chirality of an individual chiral carbon centre is indicated by the label R or S according to the rules defined by R. S. Cahn, C. K. Ingold and V. Prelog. This R/S-concept and rules for the determination of the absolute configuration in stereochemistry is known to the person skilled in the art.
The term "consisting of" as used according to the present invention means that the total amount of compound of formula (II-A) and compound of formula (II-B) or a ketal thereof and one chiral compound of formula (II-C) ideally sum up to 100 wt.-%. It is, however, not excluded that small amount of impurities or non-reactive additives may be present in amounts of less than 10 wt.-%, preferably less than 5 wt.-%, relative to the sum of compound of formula (II-A) and compound of formula (II-B) or a ketal thereof and one chiral compound of formula (II-C).
The residue $R^5$ represents either a linear or branched completely saturated $C_{6-25}$-alkyl group or a linear or branched $C_{6-25}$-alkyl group comprising at least one carbon-carbon double bond.
Preferably the group $R^5$ is of formula (III).

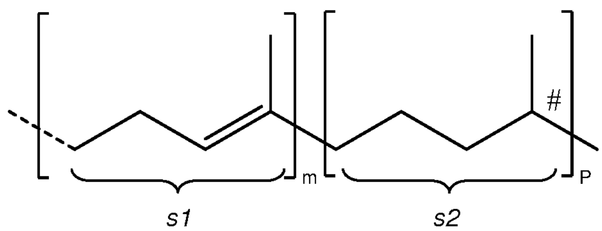

(III)

In formula (III) m and p stand independently from each other for a value of 0 to 5 provided that the sum of m and p is 1 to 5. Furthermore, the substructures in formula (III) represented by *s1* and *s2* can be in any sequence. The dotted line represents the bond by which the substituent of formula (III) is bound to the rest of the compound of formula (II-B) or formula (I). Furthermore, # represents a chiral centre, obviously except in case where said centre is linked to two methyl groups.
It is preferred that group $R^5$ is of formula (III-x).

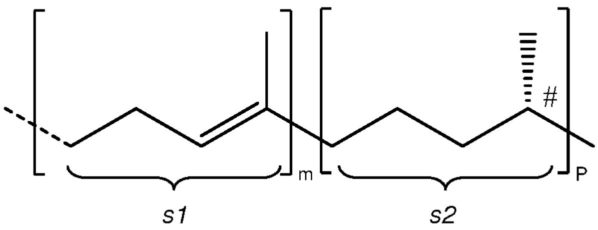

(III-x)

As mentioned above the substructures in formula (III) represented by *s1* and *s2* can be in any sequence. It is, therefore, obvious that in case that the terminal group is having the substructure *s2*, this terminal substructure has no chiral centre.

In one preferred embodiment m stands for 3 and p for 0.

In another preferred embodiment p stands for 3 and m for 0.

Therefore, $R^5$ is preferably of formula (III-A), particularly (III-ARR), or (III-B).

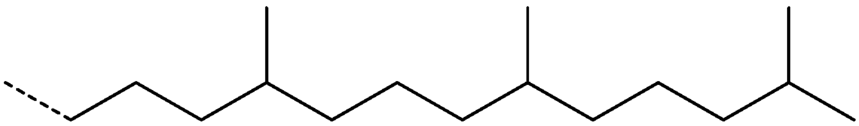

(III-A)

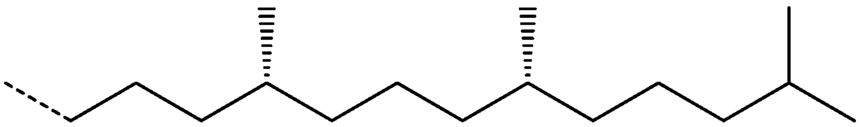

(III-ARR)

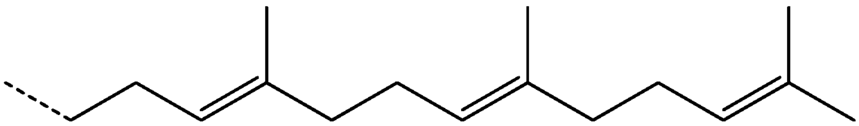

(III-B)

Preferred are the following combinations of $R^1$, $R^3$ and $R^4$:

$R^1 = R^3 = R^4 = CH_3$

or

$R^1 = R^4 = CH_3$, $R^3 = H$

or

$R^1 = H$, $R^3 = R^4 = CH_3$

or

$R^1 = R^3 = H$, $R^4 = CH_3$

$R^2$ and $R^2$ represents either hydrogen or a phenol protection group.

A phenol protection group is a group which protects the phenolic group (OH in formula (I) or (II-A)) and can be deprotected easily, i.e. by state-of-the-art methods, to the phenolic group again.

The phenol protection group forms with the rest of the molecule a chemical functionality which is particularly selected from the group consisting of ester, ether or acetal. The protection group can be easily removed by standard methods known to the person skilled in the art.

In case where the phenol protection group forms with the rest of the molecule an ether, the substituent $R^2$ or $R^2$ is particularly a linear or branched $C_{1-10}$- alkyl or cycloalkyl or aralkyl group. Preferably the substituent $R^2$ or $R^2$ is a

benzyl group or a substituted benzyl group, particularly preferred a benzyl group.

In case where the phenol protection group forms with the rest of the molecule an ester, the ester is an ester of an organic or inorganic acid.

If the ester is an ester of an organic acid, the organic acid can be a monocarboxylic acid or a polycarboxylic acid, i.e. an acid having two or more COOH-groups. Polycarboxylic acids are preferably malonic acid, succinic acid, glutaric acid, adipic acid, maleic acid or fumaric acid.

Preferably the organic acid is a monocarboxylic acid.

Hence, the substituent $R^2$ or $R^{2'}$ is preferably an acyl group. The acyl group is particularly a $C_{1-7}$-acyl, preferably acetyl, trifluoroacetyl, propionyl or benzoyl group, or a substituted benzoyl group.

If the ester is an ester of an inorganic acid, the inorganic acid is preferably nitric acid or a polyprotic acid, i.e. an acid able to donate more than one proton per acid molecule, particularly selected from the group consisting of phosphoric acid, pyrophosphoric acid, phosphorous acid, sulphuric acid and sulphurous acid.

In case where the phenol protection group forms with the rest of the molecule an acetal, the substituent $R^2$ or $R^2$ is preferably

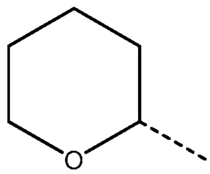 or 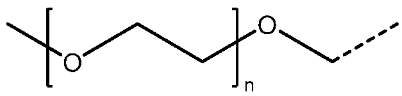

with n=0 or 1.

Hence, the acetals formed so are preferably methoxymethyl ether (MOM-ether), β-methoxyethoxymethyl ether (MEM-ether) or tetrahydropyranyl ether (THP-ether). The protection group can easily be removed by acid.

The protecting group is introduced by reaction of the corresponding molecule having an $R^2$ resp. $R^{2'}$ being H with a protecting agent.

The protecting agents leading to the corresponding phenol protection groups are known to the person skilled in the art, as well as the chemical process and conditions for this reaction. If, for example, the phenol protection group forms with the rest of the molecule an ester, the suitable protecting agent is for example an acid, an anhydride or an acyl halide.

In the case that an ester is formed by the above reaction with the protecting agent, and that said ester is an ester of an organic polycarboxylic acid or an inorganic polyprotic acid, not necessarily all acid groups are esterified to qualify as protected in the sense of this document. Preferable esters of inorganic polyprotic acids are phosphates.

It is preferred that the protection group $R^2$ resp. $R^{2'}$ is a benzoyl group or a $C_{1-4}$-acyl group, particularly acetyl or trifluoroacetyl group. The molecules in which $R^2$ resp. $R^{2'}$ represents an acyl group, particularly an acetyl group, can be easily prepared from the corresponding unprotected molecule by esterification, respectively the phenolic compound can be obtained from the corresponding ester by ester hydrolysis.

It is important to realize that the step of reacting with the protecting agent can occur at different stages of manufacture of compound of formula (I) or of formula (V), the preparation of which is described later in this document in more detail, i.e. the reaction can occur for example at the level of compound of formula (II-A) or before or after preparation of compound (I) or compound (V).

It is particularly preferred that $R^2$ and $R^{2'}$ is H.

The process of the present invention comprises the steps of reacting compound of formula (II-A) and compound of formula (II-B) or a ketal thereof.

The corresponding compounds of (II-A) and compound of formula (II-B) or a ketal thereof are easily accessible. For example compounds of (II-A) can be synthesized from the method disclosed in G. Manecke, G. Bourwieg, Chem. Ber. 1962, 95, 1413-1416.

The composition comprises at least one chiral compound of formula (II-C).

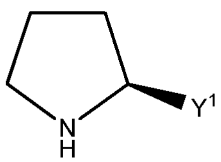 (II-C)

The group $Y^1$ represents either $CH_2Y^2$ or

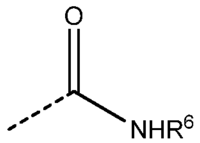

$R^6$ represents in first instance a linear or branched $C_{1-12}$-alkyl group. Particularly suitable linear or branched $C_{1-12}$-alkyl groups are methyl, ethyl, *n*-propyl, *iso*-propyl, *n*-butyl, *iso*-butyl, *sec*-butyl, *tert*-butyl, pentyl, hexyl, heptyl and octyl groups.

$R^6$ represents in second instance a linear or branched $C_{1-12}$-alkyl group which comprises further at least one aromatic group and/or C=O and/or NH and/or $NH_2$ group. Examples of suitable compounds of formula (II-C) for this embodiment are

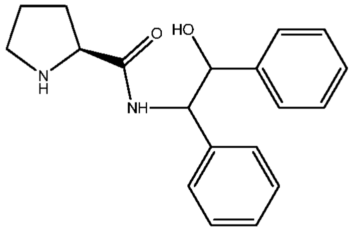
,
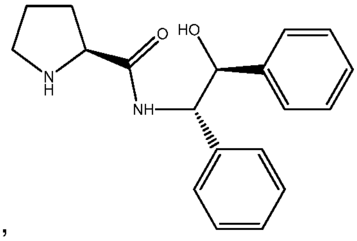
,
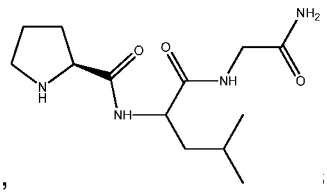
;

and

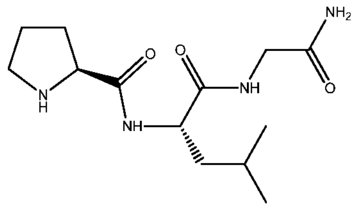

$Y^2$ represents either OH or $OR^7$ or $NHR^7$ or $NHCOOR^7$ or

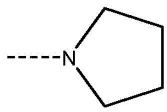

$R^7$ represents in a first instance a linear or branched $C_{1-12}$-alkyl group. Particularly suitable linear or branched $C_{1-12}$-alkyl groups are methyl, ethyl, *n*-propyl, *iso*-propyl, *n*-butyl, *iso*-butyl, *sec*-butyl, *tert*-butyl, pentyl, hexyl, heptyl and octyl groups.

$R^7$ represents in a second instance a linear or branched $C_{1-12}$-alkyl group which further comprises at least one aromatic group and/or C=O and/or NH and/or $NH_2$ group.

$R^7$ represents in a third instance an aryl group or a substituted aryl group or a heteroaryl group or a substituted heteroaryl group. The aryl group or a substituted aryl group or a heteroaryl group or a substituted heteroaryl group is particularly

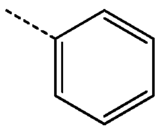
,
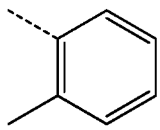
,
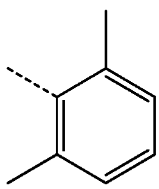
,
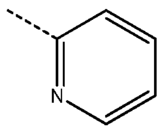
or
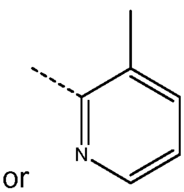

It is preferred that the compound of formula (II-C) is selected from the group consisting of

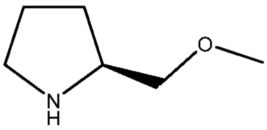, 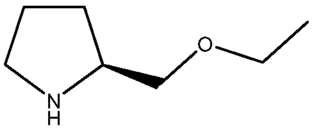, 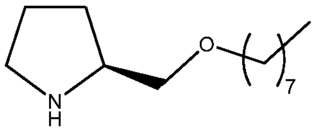,

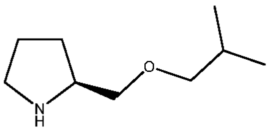, 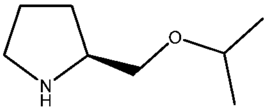, 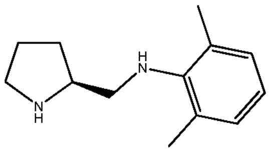, 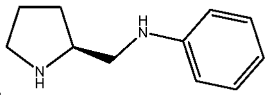,

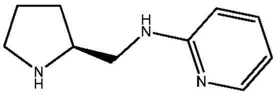, 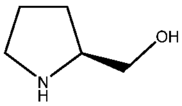, 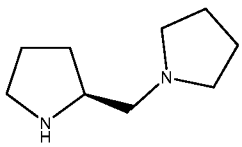, 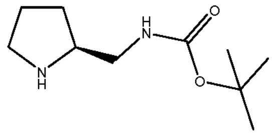,

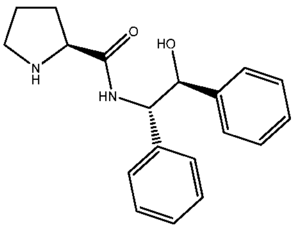, 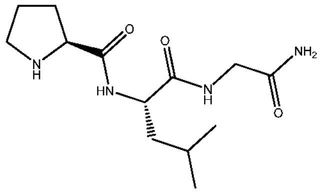 and 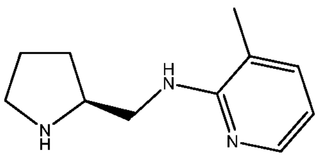

The compounds of formula (II-B) can be synthesized from corresponding precursors, for example the compound (E,E)-farnesylacetone from nerolidol by a chain-elongation reaction, as described in WO 2009/019132.

In one preferred embodiment the group $R^5$ does not comprise any chiral centres. The compound of formula (II-B) is preferred from the group consisting of (E)-6,10-dimethylundeca-5,9-dien-2-one, (5E,9E)-6,10,14-trimethylpentadeca-5,9,13-trien-2-one and (5E,9E,13E)-6,10,14,18-tetramethylnonadeca-5,9,13,17-tetraen-2-one, particularly (5E,9E)-6,10,14-trimethylpentadeca-5,9,13-trien-2-one.

When the group $R^5$ comprises chiral centres, it is preferred that the compound of formula (II-B) is in a form of pure chiral isomers.

This can be either achieved by stereospecific synthesis routes or by isolation of naturally occurring compounds or derived thereof or by separation from a mixture of the chiral stereoisomers.

For example (6R,10R)-6,10,14-trimethylpentadecan-2-one can be obtained from naturally occurring (R,R)-phytol by oxidation with $NaIO_4$ and a catalytic amount of $RuCl_3$ as disclosed by Thomas Eltz et al. in J. Chem. Ecol. (2010) 36:1322-1326.

In another preferred embodiment the compound of formula (II-B) is a methyl ketone having at least a carbon-carbon double bond in the $\gamma,\delta$-position to the keto group. Preferably it is selected from the group consisting of 6-methylhept-5-en-2-one, (E)-6,10-dimethylundec-5-en-2-one, (Z)-6,10-dimethylundec-5-en-2-one, (E)-6,10-dimethylundeca-5,9-dien-2-one, (Z)-6,10-dimethylundeca-5,9-dien-2-one, (E)-6,10,14-trimethylpentadec-5-en-2-one, (Z)-6,10,14-trimethylpentadec-5-en-2-one; (5E,9E)-6,10,14-trimethylpentadeca-5,9-dien-2-one, (5E,9Z)-6,10,14-trimethylpentadeca-5,9-dien-2-one, (5Z,9E)-6,10,14-trimethylpentadeca-5,9-dien-2-one, (5Z,9Z)-6,10,14-trimethylpentadeca-5,9-dien-2-one; (E)-6,10,14-trimethylpentadeca-5,13-dien-2-one, (Z)-6,10,14-trimethylpentadeca-5,13-dien-2-one; (5E,9E)-6,10,14-trimethylpentadeca-5,9,13-trien-2-one, (5E,9Z)-6,10,14-trimethylpentadeca-5,9,13-trien-2-one, (5Z,9E)-6,10,14-trimethylpentadeca-5,9,13-trien-2-one, (5Z,9Z)-6,10,14-trimethylpentadeca-5,9,13-trien-2-one; (E)-6,10,14,18-tetramethylnonadec-5-en-2-one, (Z)-6,10,14,18-tetramethylnonadec-5-en-2-one; (5E,9E)-6,10,14,18-tetramethylnonadeca-5,9-dien-2-one, (5E,9Z)-6,10,14,18-tetramethylnonadeca-5,9-dien-2-one, (5Z,9E)-6,10,14,18-tetra-methylnonadeca-5,9-dien-2-one, (5Z,9Z)-6,10,14,18-tetramethylnonadeca-5,9-dien-2-one; (5E,13E)-6,10,14,18-tetramethylnonadeca-5,13-dien-2-one, (5E,13Z)-6,10,14,18-tetramethylnonadeca-5,13-dien-2-one, (5Z,13E)-6,10,14,18-tetra-methylnonadeca-5,13-dien-2-one, (5Z,13Z)-6,10,14,18-tetramethylnonadeca-5,13-dien-2-one; (E)-6,10,14,18-tetramethylnonadeca-5,17-dien-2-one, (Z)-6,10,14,18-tetramethylnonadeca-5,17-dien-2-one; (5E,9E,13E)-6,10,14,18-tetramethyl-nonadeca-5,9,13-trien-2-one, (5E,9E,13Z)-6,10,14,18-tetramethylnonadeca-5,9,13-trien-2-one, (5E,9Z,13E)-6,10,14,18-tetramethylnonadeca-5,9,13-trien-2-one, (5E,9Z,13Z)-6,10,14,18-tetramethylnonadeca-5,9,13-trien-2-one, (5Z,9E,13E)-6,10,14,18-tetramethylnonadeca-5,9,13-trien-2-one, (5Z,9E,13Z)-6,10,14,18-tetramethylnonadeca-5,9,13-trien-2-one, (5Z,9Z,13E)-6,10,14,18-tetra-methylnonadeca-5,9,13-trien-2-one, (5Z,9Z,13Z)-6,10,14,18-tetramethylnona-deca-5,9,13-trien-2-one; (5E,13E)-6,10,14,18-tetramethylnonadeca-5,13,17-trien-2-one, (5E,13Z)-6,10,14,18-tetramethyl-nonadeca-5,13,17-trien-2-one, (5Z,13E)-6,10,14,18-tetramethylnonadeca-5,13,17-trien-2-one, (5Z,13Z)-6,10,14,18-tetra-methylnonadeca-5,13,17-trien-2-one; (5E,9E)-6,10,14,18-tetramethylnonadeca-5,9,17-trien-2-one, (5E,9Z)-6,10,14,18-tetramethylnonadeca-5,9,17-trien-2-one, (5Z,9E)-6,10,14,18-tetramethylnonadeca-5,9,17-trien-2-one, (5Z,9Z)-6,10,14,18-tetramethylnonadeca-5,9,17-trien-2-one; (5E,9E,13E)-6,10,14,18-tetramethyl-nonadeca-5,9,13,17-tetraen-2-one, (5E,9E,13Z)-6,10,14,18-tetramethylnonadeca-5,9,13,17-tetraen-2-one, (5E,9Z,13E)-6,10,14,18-tetramethylnonadeca-5,9,13,17-tetraen-2-one, (5E,9Z,13Z)-6,10,14,18-tetramethylnonadeca-5,9,13,17-tetraen-2-one, (ZE,9E,13E)-6,10,14,18-tetramethylnonadeca-5,9,13,17-tetraen-2-one, (5Z,9E, 13Z)-6,10,14,18-tetramethylnonadeca-5,9,13,17-tetraen-2-one, (5Z,9Z,13E)-6,10,14,18-tetramethylnonadeca-5,9,13,17-tetraen-2-one, (5Z,9Z,13Z)-6,10,14,18-tetramethyl nonadeca-5,9,13,17-tetraen-2-one, (5E,9E,13E)-6,10,14,18-tetramethylnonadeca-5,9,13-trien-2-one.

In case there are chiral centres in the group $R^5$, particularly if $R^5$ has the formula (III-ARR), the corresponding compounds of formula (II-B) can be prepared by asymmetrically hydrogenating olefinic unsaturated precursors thereof using chiral iridium complexes as disclosed in WO 2006/066863 A1 and WO 2012/152779 A1 the entire content of which is hereby incorporated by reference.

In case the compounds just mentioned have chiral carbon centre(s) it is preferred that said chiral centre(s) has/have the configuration as indicated in formula (III-x).

Preferably the compound of formula (II-B) in this embodiment is (E)-6,10-dimethylundec-5,9-dien-2-one (geranyl acetone) or (Z)-6,10-dimethylundec-5,9-dien-2-one (neryl acetone) or (5E,9E)-6,10,14-trimethylpentadeca-5,9-dien-2-one (E,E-farnesylacetone) or (5Z,9Z)-6,10,14-trimethylpentadeca-5,9-dien-2-one (Z,Z-farnesylacetone) or (E)-6,10-dimethylundec-5-en-2-one or (Z)-6,10-dimethyl-undec-5-en-2-one or (E)-6,10,14-trimethylpentadec-5-en-2-one or (Z)-6,10,14-trimethylpentadec-5-en-2-one, preferably geranyl acetone or E,E-farnesylacetone or (Z)-6,10-dimethylundec-5-en-2-one or (Z)-6,10,14-trimethylpentadec-5-en-2-one, more preferably geranyl acetone or E,E-farnesylacetone.

More preferred the compound of formula (II-B) is 6,10-dimethylundecan-2-one or 6,10,14-trimethylpentadecan-2-one.

Most preferred the compound of formula (II-B) is either (6R),10-dimethylundecan-2-one or (6R,10R),14-trimethylpentadecan-2-one.

In one embodiment a compound of formula (II-A) is reacted with a ketal of a compound, i.e. a ketone, of formula (II-B) in the presence of at least one chiral compound of formula (II-C).

The formation of a ketal from a ketone, per se, is known to the person skilled in the art.

The ketal of a ketone of formula (II-B) can be preferably formed from the ketone of formula (II-B) and an alcohol.

It is known to the person skilled in the art that there are alternative routes of synthesis for ketals. In principle, the ketal can also be formed by treating a ketone with ortho-esters or by trans-ketalization such as disclosed for example in Pério et al., Tetrahedron Letters 1997, 38 (45), 7867-7870, or in Lorette and Howard, J. Org. Chem. 1960, 521-525, the entire content of both is hereby incorporated by reference.

Preferably the ketal is formed from the above mentioned ketone of formula (II-B) and an alcohol.

The alcohol can comprise one or more hydroxyl groups. The alcohol may be a phenolic alcohol or an aliphatic or cycloaliphatic alcohol. Preferably the alcohol has one or two hydroxyl groups.

In case the alcohol has one hydroxyl group, the alcohol is preferably an alcohol which has 1 to 12 carbon atoms. Particularly, the alcohol having one hydroxyl group is selected from the group consisting of methanol, ethanol, 1-propanol, 2-propanol, 1-butanol, 2-methyl-1-propanol, 2-butanol, pentane-1-ol, 3-methylbutane-1-ol, 2-methylbutane-1-ol, 2,2-dimethylpropan-1-ol, pentane-3-ol, pentane-2-ol, 3-methylbutane-2-ol, 2-methylbutan-2-ol, hexane-1-ol, hexane-2-ol, hexane-3-ol, 2-methyl-1-pentanol, 3-methyl-1-pentanol, 4-methyl-1-pentanol, 3-methyl-2-pentanol, 4-methyl-2-pentanol, 2-methyl-3-pentanol, 2,2-dimethyl-1-butanol, 2,3-dimethyl-1-butanol, 3,3-dimethyl-1-butanol, 3,3-dimethyl-2-butanol, 2-ethyl-1-butanol, and all structural isomers of heptanol, octanol and halogenated $C_{1-8}$-alkyl alcohols, particularly 2,2,2-trifluoroethanol. Particularly suitable are primary or secondary alcohols. Preferably primary alcohols are used as alcohols with one hydroxyl group.

In another embodiment the alcohol is a diol, hence has two hydroxyl groups. Preferably the diol is selected from the group consisting of ethane-1,2-diol, propane-1,2-diol, propane-1,3-diol, butane-1,4-diol, butane-1,3-diol, butane-1,2-diol, butane-2,3-diol, 2-methylpropane-1,2-diol, 2,2-dimethylpropane-1,3-diol, 1,2-dimethylpropane-1,3-diol, benzene-1,2-diol and cyclohexane-1,2-diols. From two cyclohexane-1,2-diols the preferred stereoisomer is syn-cyclohexane-1,2-diol (=*cis*-cyclohexane-1,2-diol).

The two hydroxyl group are in one embodiment bound to two adjacent carbon atoms, hence these diols are vicinal diols. Vicinal diols form a 5 membered ring in a ketal.

Particularly suitable are vicinal diols which are selected from the group consisting of ethane-1,2-diol, propane-1,2-diol, butane-1,2-diol, butane-2,3-diol, 2-methylpropane-1,2-diol, benzene-1,2-diol and *syn*-cyclohexane-1,2-diol,

particularly ethane-1,2-diol.

Other particularly suitable are diols, in which the hydroxyl groups are separated by 3 carbon atoms, and, hence, form a very stable 6 membered ring in a ketal. Particularly suitable diols of this type are propane-1,3-diol, butane-1,3-diol, 2-methylpropane-1,3-diol, 2-methylbutane-1,3-diol, 2,2-dimethylpropane-1,3-diol, 1,2-dimethylpropane-1,3-diol, 3-methylpentane-2,4-diol and 2-(hydroxymethyl)-cyclohexanol.

Preferably primary alcohols are used as diols.

The reaction conditions and stoichiometry used for the ketal formation are known to the person skilled in the art.

It is more preferred that the compound of formula (II-C) is selected from the group consisting of

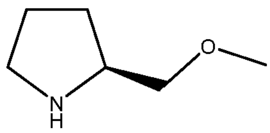,
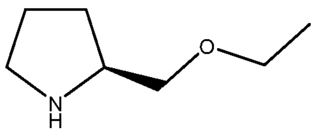,
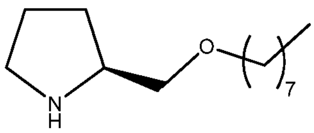,

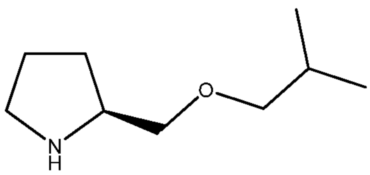,
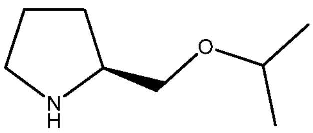,
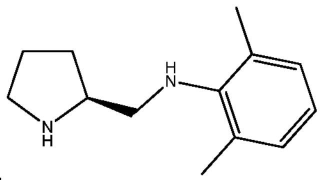,

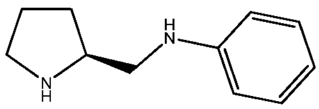 and 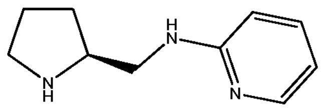

The compounds of formula (II-C) are chiral compounds. The compounds are either used directly as pure stereoisomers or separated by known techniques into the R- and the S-stereoisomer prior to the use for the present invention.

It has been found that the isomer shown in formula (II-C) yields preferentially the isomers of compound of formula (I), respectively of formula (V), showing the R-configuration at the chiral centre indicated by *.

Therefore, it has been found that the chirality of the compound of formula (II-C) has an important effect on the chirality of the compound being formed, i.e. on compound of formula (I) or of formula (V).

Hence, the isomer having the R-configuration at the chiral centre marked by * in formula (I) is preferentially formed in respect to the corresponding isomer having the S-configuration at said chiral centre by the above process.

On the other hand, it has been found that when using the stereoisomers shown in formula (II-C') instead of compounds of formula (II-C) preferentially the isomers of compound of formula (I) resp. formula (V) showing the S-configuration at the chiral centre indicated by * are obtained.

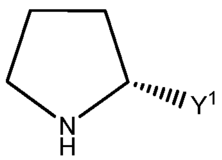 (II-C')

Compound of formula (II-A) and compound of formula (II-B) or a ketal thereof are reacted in the presence of at least one chiral compound of formula (II-C).

The composition consisting of compound of formula (II-A), compound of formula (II-B) or a ketal thereof and at least one chiral compound of formula (II-C) ideally sum up to 100 wt.-%, however, may comprise also small amount of impurities or non-reactive additives may be present in amounts of less than 10 wt.-%, preferably less than 5 wt.-%, relative to the sum of compound of formula (II-A) and compound of formula (II-B) or a ketal thereof and one chiral compound of formula (II-C).

This reaction occurs preferably in neat. The temperature is preferably between 80°C and 150°C, more preferably of between 90°C and 140°C, most preferably at a temperature of between 100 and 110°C at ambient pressure.

It has been found that the lower the temperature for the reaction of compound of formula (II-A) and compound of formula (II-B) or a ketal thereof is, the higher the chiral purity of the compound of formula (I) resp. (V) in view of chirality

at the chiral centre indicated by * is. This chiral purity is expressed by the enantiomeric excess (ee) being determined by the absolute value of the difference of amounts of the R and S isomers divided by the sum of amounts of both isomers: and is normally expressed in %.

$$ee = abs\left(\frac{[R]-[S]}{[R]+[S]}\right)$$

We have been able to show that by using a reaction temperature of 0°C the process has yielded in the formation of a product having an enantiomeric excess up to 40%, corresponding to a ratio of [R]/[S] of 2.3. However, the reaction rate was rather low.

In view of reaction rate, it is preferred to have the reaction taking place at higher temperatures higher than 0°C.

Furthermore, it might be helpful, particularly in the case where at low reaction temperatures are used, to use molecular sieves in the reaction medium.

The enantiomeric ratio can be increased further by optimizing the reaction conditions. The larger the enantiomeric ratio is the better. However, also at lower enantiomeric ratios the invention can be advantageous as the complete separation of the isomers, such as by chromatography, particularly by chromatography using chiral stationary phases, needs much less efforts as compared to a racemic mixture. Hence, the enantiomeric ratio should be at least 15 %, preferably at least 20 %, more preferably at least 25 %.

In a further aspect, the invention relates to a process of manufacturing a compound of formula (V) comprising the steps

i) process of manufacturing of formula (I) as it has been described in detail above;
ii) reducing of compound of formula (I)

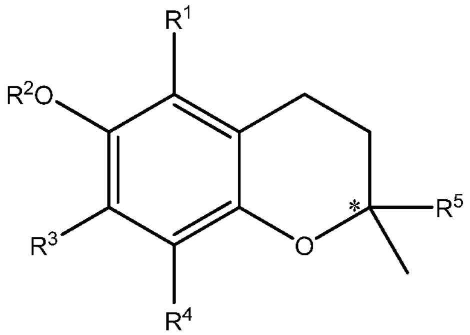

(V).

The substituents $R^1$, $R^2$, $R^3$, $R^4$ and $R^5$ are already discussed in detail above.

Most preferably the chiral isomers of formula (V) are the isomers selected from the group consisting of

- $\alpha$-Tocopherol ($R^1 = R^3 = R^4 = CH_3$, $R^5$ = (II-A), particularly (II-ARR), $R^2$ = H),
- $\beta$-Tocopherol ($R^1 = R^4 = CH_3$, $R^3$ = H, $R^5$ = (II-A), particularly (II-ARR), $R^2$ = H),
- $\gamma$-Tocopherol ($R^1$ = H, $R^3 = R^4 = CH_3$, $R^5$ = (II-A), particularly (II-ARR), $R^2$ = H),
- $\delta$-Tocopherol ($R^1 = R^3$ = H, $R^4 = CH_3$, $R^5$ = (II-A), particularly (II-ARR), $R^2$ = H),
- $\alpha$-Tocotrienol ($R^1 = R^3 = R^4 = CH_3$, $R^5$ = (II-B), $R^2$ = H),
- $\beta$-Tocotrienol ($R^1 = R^4 = CH_3$, $R^3$ = H, $R^5$ = (II-B), $R^2$ = H),
- $\gamma$-Tocotrienol ($R^1$ = H, $R^3 = R^4 = CH_3$, $R^5$ = (II-B), $R^2$ = H),
- $\delta$-Tocotrieno ($R^1 = R^3$ = H, $R^4 = CH_3$, $R^5$ = (II-B), $R^2$ = H),

and the esters, particularly the acetates ($R^2 = COCH_3$), or phosphates thereof.

Particularly preferred compounds of formula (V) are esters of organic and inorganic acids. Examples of esters of organic acids are acetate and succinate esters, esters of inorganic esters are tocopheryl phosphates, ditocopheryl phosphates, particularly $\alpha$-tocopheryl phosphate and $\alpha$-ditocopheryl phosphate.

Most preferred compounds of formula (V) are tocopherols and tocopheryl acetates.

The reduction in step ii) can be made by different ways. Typically it is reduced by using a reduction means.

Preferably the reduction is made by metallic zinc in the presence of an acid or an acid mixture, for example as disclosed for in US 6,096,907 or EP 0 989 126 the whole disclosure of which is incorporated herein by reference.

The reduction step ii) is typically done in stirred vessel under inert atmosphere. It is further preferred that the

step ii) is done at a temperature in the range of 30 to 90°C, particularly between 40 and 65°C.

After completion of the reaction the compound of formula (V) is purified, particularly by means of extraction.

It has been observed that the reduction of compound of formula (I) to compound of formula (V) does not modify the chirality of the chiral centre indicated by * in the formulae (I) resp. (V).

It has been found that the isomer shown in formula (II-C) yields preferentially the isomers of compound of formula (I), respectively of formula (V), showing the R-configuration at the chiral centre indicated by *.

Hence, the isomer having the R-configuration at the chiral centre marked by * in formula (V) is preferentially formed in respect to the corresponding isomer having the S-configuration at said chiral centre.

On the other hand, it has been found that when using the stereoisomers shown in formula (II-C') instead of compounds of formula (II-C) preferentially the isomers of compound of formula (I) resp. formula (V) showing the S-configuration at the chiral centre indicated by * are obtained.

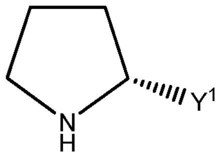

(II-C')

In a further aspect, the invention relates to a composition consisting of

a) at least one compound of formula (II-A) and
b) at least one ketone of formula (II-B) or a ketal thereof and
c) at least one chiral compound of formula (II-C)

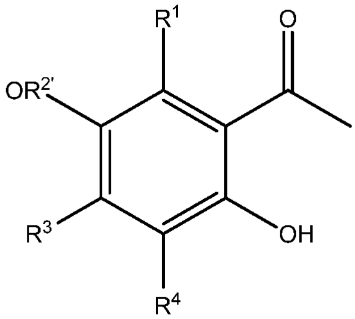

(II-A)

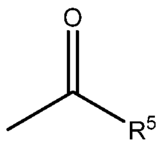

(II-B)

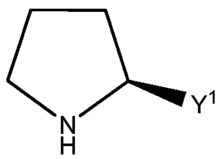

(II-C).

The substituents $R^1$, $R^2$, $R^3$, $R^4$, $R^5$ and $Y^1$ have are already been discussed in detail above.

Furthermore, details for the compound of formula (II-A), for compound of formula (II-B) or a ketal thereof and for chiral compound of formula (II-C) as well their preferred embodiments and their ratios have been discussed in detail already above.

As described above this composition is very suitable for the synthesis of compound of formula (I) which can be transformed to compound of formula (V).

Therefore, a chiral compound of formula (II-C) can be used for the preparation of tocopherols or tocotrienols as it also discussed in great detail above. This use particularly involves the use of of a chiral compound of formula (II-C) for the preparation of compound of formula (I) followed by transformation to compound of formula (V). When the reaction is done in a composition consisting of compound of formula (II-A) and a ketone of formula (II-B) or a ketal thereof

and a chiral compound of formula (II-C) the formation of the stereoisomer of formula (I) resp. (V) having the R configuration at the chiral carbon centre marked by * in formula (I) resp. (V) is obtained in an excess related to the corresponding stereoisomer having the S-configuration.

The details for chiral compound of formula (II-C), for compound of formula (I) and for formula (V) as well their preferred embodiments and their ratios have been discussed in detail already above.

**Examples**

The present invention is further illustrated by the following experiments.

Use of different bases

0.5 mmol (corresponding to 100 mg) of 2-acetyl-3,5,6-trimethylhydroquinone has been added in a 20 mL round bottom flask equipped with a magnetic stirring bar, heating device, water separator and argon supply at 23°C. Then 0.514 mmol (corresponding to 134 mg) of E,E-farnesylacetone has been is added and for example ***1*** finally 0.795 mmol of the base indicated in table 1 has been added. In case of example ***Ref.* 3** 2.5 mL (corresponding to 2.2 g) of toluene have been added into the flask before adding the E,E-farnesylacetone.

The corresponding reaction mixture has been stirred during 16 hours at 23°C. While heating to 120 °C water is distilled off and the reaction mixture was getting brown. After the indicated time, the reaction mixture was cooled to 23°C. Then 1 mL of 2 N HCl has been added and the mixture has been transferred to a separation funnel and was well shaken. The toluene phase was separated and washed with portions of 10 mL water until a neutral water phase was obtained. The organic layers are dried over sodium sulfate, filtered and concentrated at 40°C and 10 mbar.

The product formed and isolated by column chromatography on $SiO_2$ has been identified to be 6-hydroxy-2,5,7,8-tetramethyl-2-((3E,7E)-4,8,12-trimethyltrideca-3,7,11-trien-1-yl)chroman-4-one:

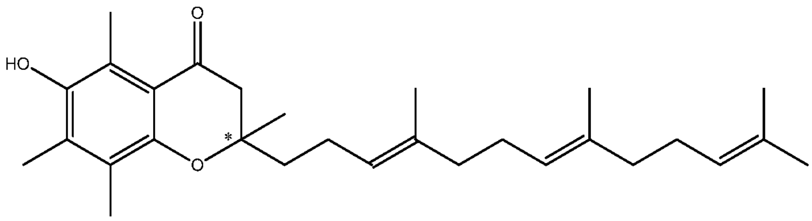

$^1H$ NMR ($CDCl_3$, 300 MHz) $\delta$ 1.30 (s, 3H); 1.51 (s, 6H); 1.52 (s, 3H); 1.54-1.58 (m, 1 H); 1.61 (d, J= 0.9 Hz, 3H); 1.67-1.78 (m, 1 H); 1.67-2.10 (m, 10H); 2.08 (s, 3H); 2.16 (s, 3H); 2.48 (s, 3H); 2.51 (d, J=15.8 Hz, 1 H); 2.68 (d, J=15.8Hz, 1 H), 4.45 (s br, 1 H); 4.99-5.05 (m, 3H) ppm.

$^{13}C$ NMR ($CDCl_3$, 75.5 MHz) $\delta$ 12.1; 12.8; 13.3; 15.9; 16.0; 17.7; 22.2; 23.7; 25.1; 26.6; 26.8; 39.4; 39.7 (2C); 49.5; 79.4; 116.7; 120.4; 123.5; 124.0; 124.1; 124.4; 131.3; 132.0; 135.1; 135.7; 145.8; 152.8; 195.2 ppm.

Determination of enantiomeric ratio: HPLC, Chiralcel® OD-H, 250×4.6 mm, 10 mL EtOH, 990 mL n-hexane, 1.0 mL/ min; detection at 220 nm.

Table 1: Different compositions.

| | **Base** | Further component | Yield[1] [%] | [R]: [S] | ee [%] |
|---|---|---|---|---|---|
| ***Ref.1*** | none | - | 0 | 2 | 2 |
| ***Ref.2*** | pyrrolidine | - | n.d.[3] | 50:50 | 0 |
| ***Ref.3*** | (S)-2-(methoxymethyl)pyrrolidine | toluene | 2.2 | 50:50 | 0 |
| ***1*** | (S)-2-(methoxymethyl)pyrrolidine | - | 26 | 63:37 | 26 |
| ***2*** | Pro-Leu-Gly amide[4] | - | 13 | 56:44 | 12 |

[1] yield relative to 2-acetyl-3,5,6-trimethylhydroquinone
[2] as no reaction occurred (yield: 0 %) no measurements were possible
[3] n.d. = not determined
[4] Pro-Leu-Gly amide = L-Proline-L-leucine-glycine-amide = (2S)-N-[(2S)-1-[(2-amino-2-oxoethyl)amino]-4-methyl-1-oxopentan-2-yl]pyrrolidine-2-carboxamide

Conversion of chromanones to chromans

6-hydroxy-2,5,7,8-tetramethyl-2-((3E,7E)-4,8,12-trimethyltrideca-3,7,11-trien-1-yl)chroman-4-one been transformed to α-tocotrienol by treatment with zinc dust and aqueous hydrochloric acid, as described in detail by Baldenius et al., EP 0 989 126 A1:

6-Hydroxy-2,5,7,8-tetramethyl-2-((3E,7E)-4,8,12-trimethyltrideca-3,7,11-trien-1-yl)chroman-4-one (5.0 mmol) was dissolved under an argon atmosphere in 25 mL toluene, and 25% aqueous HCl (41.7 mL, 340 mmol) was added. To this mechanically stirred two-phasic mixture zinc dust (65 mmol) was added in small portions (ca. 0.5 g) during 4 h. Stirring was continued at 40°C for 16 h and at 65°C for 1 h. After completion of the reaction (TLC control), the mixture was cooled to room temperature and filtered through a pad of Dicalite. The filter residue was washed with 100 mL n-heptane, and the combined filtrates washed with 50 mL water. The organic layer was dried over sodium sulfate, filtered, concentrated at 40°C and 10 mbar and dried for 2 h at 0.003 mbar at 23°C. The 2.22 g yellowish-brown oil was purified by column chromatography ($SiO_2$, n-hexane/ EtOAc 9:1). After evaporation (40°C/ 20 mbar) and drying (0.021 mbar/ 23°C) α-tocotrienol was obtained as a yellowish-brown oil (1.291 g, purity 93.9wt%, yield 57%).

The compound obtained showed identical retention time in comparison to an authentic sample of natural (R,E,E)-α-tocotrienol, and the values obtained by measuring the $^1H$ NMR ($CDCl_3$, 300 MHz) were identical with the values for α-tocotrienol, as for example reported by P. Schudel et al., Helv. Chim. Acta 1963, 46, 2517-2526.

Determination of enantiomeric ratio: HPLC, Chiralcel® OD-H, 250×4.6 mm, 0.5% EtOH in n-hexane, 1.0 mL/min; detection at 220 nm.

## Claims

**1.** Process for the manufacturing of a compound of formula (I)

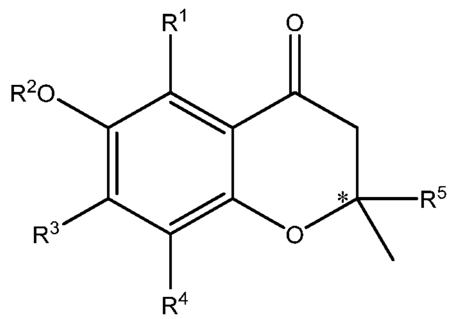

(I)

comprising the step of reacting a composition consisting of

- compound of formula (II-A);
- compound of formula (II-B) or a ketal thereof and
- at least one chiral compound of formula (II-C)

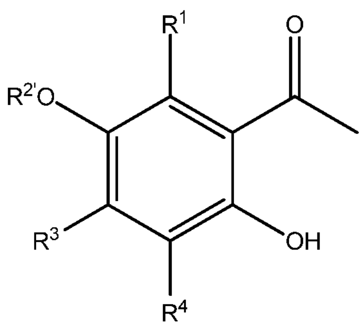

(II-A)

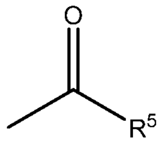

(II-B)

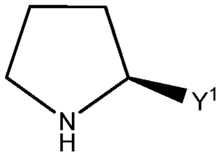

(II-C)

wherein $R^1$, $R^3$ and $R^4$ are independently from each other hydrogen or methyl groups;
$R^2$ and $R^2$ represents hydrogen or a phenol protection group;
$R^5$ represents either a linear or branched completely saturated $C_{6-25}$-alkyl group or a linear or branched $C_{6-25}$-alkyl group comprising at least one carbon-carbon double bond;
$Y^1$ represents either $CH_2Y^2$ or

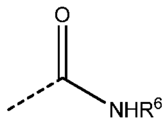

wherein $R^6$ represents a linear or branched $C_{1-12}$-alkyl group which optionally further comprises at least one aromatic group and/or C=O and/or NH and/or $NH_2$ group;
$Y^2$ represents either OH or $OR^7$ or $NHR^7$ or $NHCOOR^7$ or

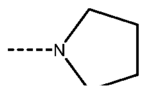

wherein $R^7$ represents either

a linear or branched $C_{1-12}$-alkyl group which optionally further comprises at least one aromatic group and/or C=O and/or NH and/or $NH_2$ group
or
an aryl group or a substituted aryl group or a heteroaryl group or a substituted heteroaryl group;

and the dotted line(s) represents the bond(s) by which the corresponding substituent is bound to the rest of formula (II-C);
and wherein * represents the chiral centre of the chiral isomer of formula (I).

2. Process according to claim 1 **characterized in that** $R^5$ is of formula (III)

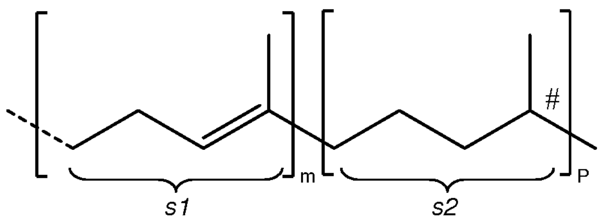

(III)

wherein m and p stand independently from each other for a value of 0 to 5 provided that the sum of m and p is 1 to 5, and where the substructures in formula (III) represented by *s1* and s2can be in any sequence;
and the dotted line represents the bond by which the substituent of formula (III) is bound to the rest of the compound of formula (II-B) or formula (I);
and wherein # represents a chiral centre, obviously except in case where said centre is linked to two methyl groups.

3. Process according to anyone of the preceding claims **characterized in that**

$R^1 = R^3 = R^4 = CH_3$

or

$R^1 = R^4 = CH_3$, $R^3 = H$

or

$R^1 = H$, $R^3 = R^4 = CH_3$

or

$R^1 = R^3 = H$, $R^4 = CH_3$.

4. Process according to anyone of the preceding claims **characterized in that** the compound of formula (II-C) is selected from the group consisting of

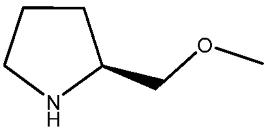 , 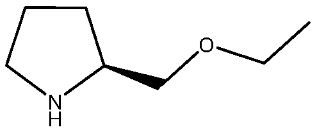 , 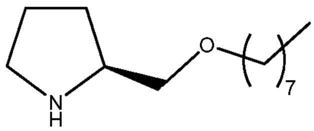 ,

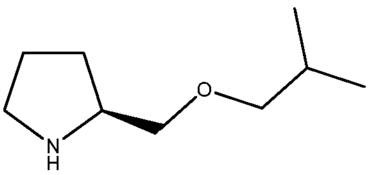 , 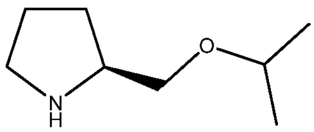 , 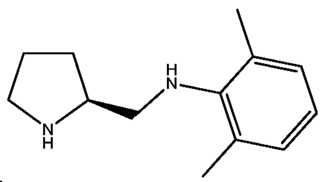 ,

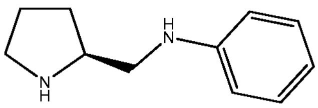 and 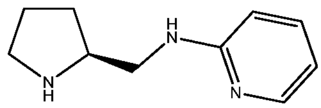

5. Process of manufacturing a compound of formula (V) comprising the steps

i) process of manufacturing of formula (I) according to anyone of the claims 1 to 4;
ii) reducing of compound of formula (I)

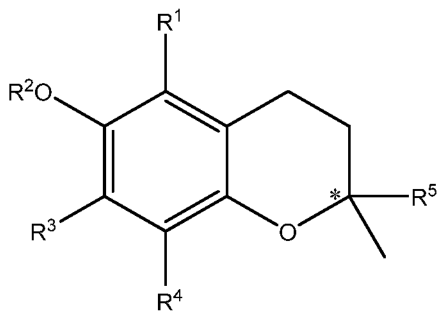 (V).

6. Process according to anyone of the claims 1 to 5, **characterized in that** the isomer having the R-configuration at the chiral centre marked by * in formula (I) or (V) is preferentially formed in respect to the corresponding isomer having the S-configuration at said chiral centre.

**7.** Composition consisting of

a) at least one compound of formula (II-A) and
b) at least one ketone of formula (II-B) or a ketal thereof and
c) at least one chiral compound of formula (II-C)

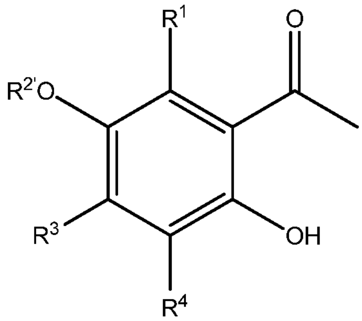

(II-A)

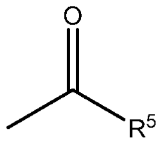

(II-B)

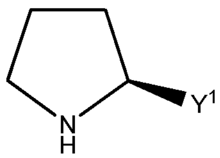

(II-C)

wherein $R^1$, $R^3$ and $R^4$ are independently from each other hydrogen or methyl groups;
$R^{2'}$ represents hydrogen or a phenol protection group;
$R^5$ represents either a linear or branched completely saturated $C_{6-25}$-alkyl group or a linear or branched $C_{6-25}$-alkyl group comprising at least one carbon-carbon double bond;
$Y^1$ represents either $CH_2Y^2$ or

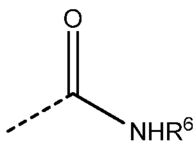

wherein $R^6$ represents a linear or branched $C_{1-12}$-alkyl group which optionally further comprises at least one aromatic group and/or C=O and/or NH and/or $NH_2$ group;
$Y^2$ represents either OH or $OR^7$ or $NHR^7$ or $NHCOOR^7$ or

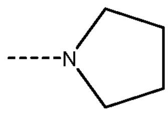

wherein $R^7$ represents either

a linear or branched $C_{1-12}$-alkyl group which optionally further comprises at least one aromatic group and/or C=O and/or NH and/or $NH_2$ group or
an aryl group or a substituted aryl group or a heteroaryl group or a substituted heteroaryl group;

and the dotted line(s) represents the bond(s) by which the corresponding substituent is bound to the rest of

formula (II-C).

**8.** Composition according to claim 7, **characterized in that** $R^5$ is of formula (III)

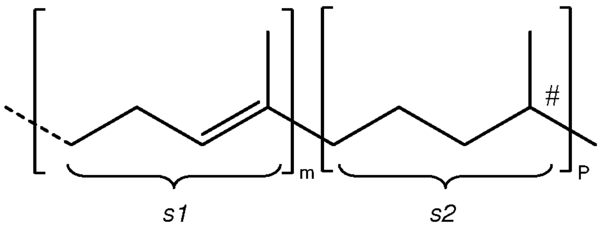

(III)

wherein m and p stand independently from each other for a value of 0 to 5 provided that the sum of m and p is 1 to 5, and where the substructures in formula (III) represented by *s1* and s2can be in any sequence;
and the dotted line represents the bond by which the substituent of formula (III) is bound to the rest of compound of formula (II-B);
and wherein # represents a chiral centre, obviously except in case where said centre is linked to two methyl groups.

**9.** Composition according to claim 7 or 8 **characterized in that** the compound of formula (II-C) is selected from the group consisting of

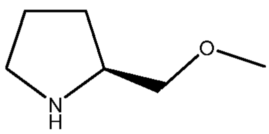
,
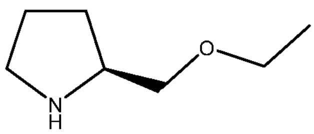
,
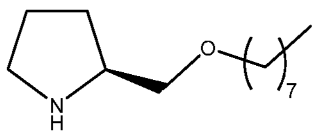
,
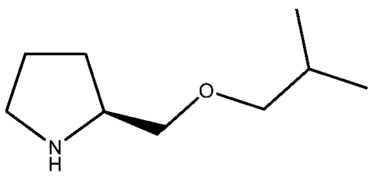
,
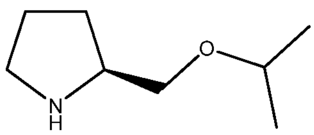
,
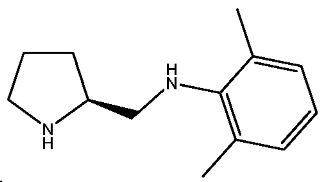
,
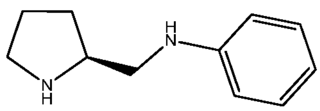
and
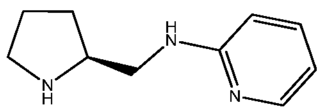

**Patentansprüche**

**1.** Verfahren zur Herstellung einer Verbindung der Formel (I)

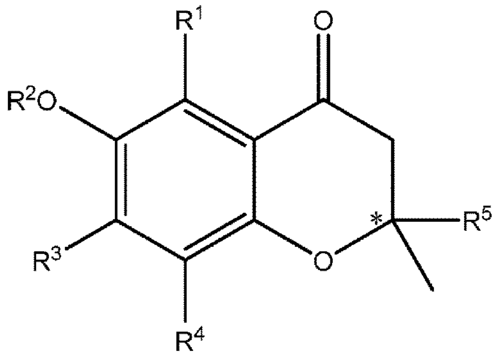

(I)

umfassend den Schritt des Umsetzens einer Zusammensetzung, bestehend aus

- einer Verbindung der Formel (II-A);
- einer Verbindung der Formel (II-B) oder einem Ketal davon und
- mindestens einer chiralen Verbindung der Formel (II-C)

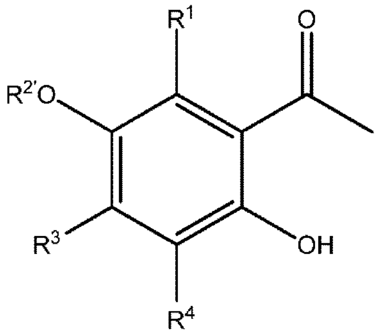

(II-A)

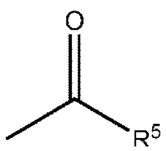

(II-B)

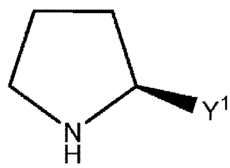

(II-C)

wobei $R^1$, $R^3$ und $R^4$ unabhängig voneinander für Wasserstoff oder Methylgruppen stehen;
$R^2$ und $R^{2'}$ für Wasserstoff oder eine Phenol-Schutzgruppe stehen;
$R^5$ entweder für eine lineare oder verzweigte vollständig gesättigte $C_{6-25}$-Alkylgruppe oder für eine lineare oder verzweigte $C_{6-25}$-Alkylgruppe mit mindestens einer Kohlenstoff-Kohlenstoff-Doppelbindung steht;
$Y^1$ entweder für $CH_2Y^2$ oder für

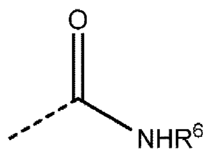

steht,

wobei $R^6$ für eine lineare oder verzweigte $C_{1-12}$-Alkylgruppe, die gegebenenfalls ferner mindestens eine aromatische Gruppe und/oder C=0- und/oder NH- und/oder $NH_2$-Gruppe umfasst, steht;
$Y^2$ entweder für OH oder für $OR^7$ oder für $NHR^7$ oder für $NHCOOR^7$ oder für

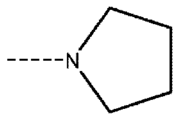

steht,
wobei $R^7$ entweder

für eine lineare oder verzweigte $C_{1-12}$-Alkylgruppe, die gegebenenfalls ferner mindestens eine aromatische Gruppe und/oder C=O-und/oder NH- und/oder $NH_2$-Gruppe umfasst, oder
für eine Arylgruppe oder eine substituierte Arylgruppe oder eine Heteroarylgruppe oder eine substituierte Heteroarylgruppe
steht;

und die gestrichelte Linie bzw. die gestrichelten Linien für die Bindung bzw. die Bindungen, über die der entsprechende Substituent an den Rest der Formel (II-C) gebunden ist, steht bzw. stehen;
und wobei * für das Chiralitätszentrum des chiralen Isomers der Formel (I) steht.

2. Verfahren nach Anspruch 1, **dadurch gekennzeichnet, dass** $R^5$ die Formel (III) aufweist:

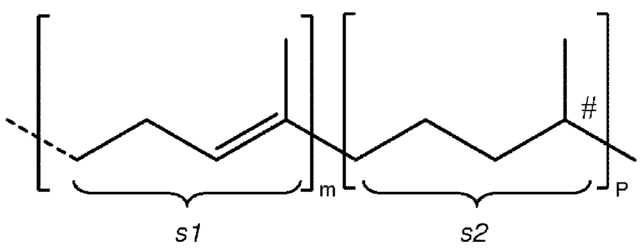

(III)

wobei m und p unabhängig voneinander für einen Wert von 0 bis 5 stehen, mit der Maßgabe, dass die Summe von m und p 1 bis 5 beträgt,
und wobei die durch *s1* und *s2* wiedergegebenen Unterstrukturen in Formel (III) in beliebiger Reihenfolge vorliegen können;
und die gestrichelte Linie für die Bindung steht, über die der Substituent der Formel (III) an den Rest der Verbindung der Formel (II-B) oder Formel (I) gebunden ist;
und wobei # für ein Chiralitätszentrum steht, offensichtlich mit Ausnahme des Falls, in dem das Zentrum an zwei Methylgruppen gebunden ist.

3. Verfahren nach einem der vorhergehenden Ansprüche, **dadurch gekennzeichnet, dass**

$$R^1 = R^3 = R^4 = CH_3$$

oder

$$R^1 = R^4 = CH_3, R^3 = H$$

oder

$$R^1 = H, R^3 = R^4 = CH_3$$

oder

$$R^1 = R^3 = H, R^4 = CH_3.$$

**4.** Verfahren nach einem der vorhergehenden Ansprüche, **dadurch gekennzeichnet, dass** die Verbindung der Formel (II-C) aus der Gruppe bestehend aus

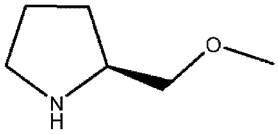 , 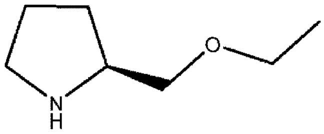 , 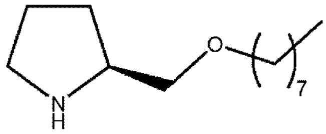 ,

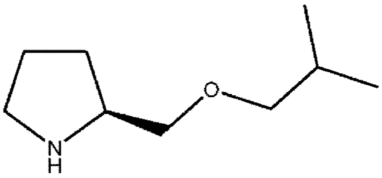 , 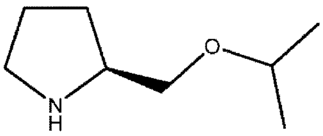 , 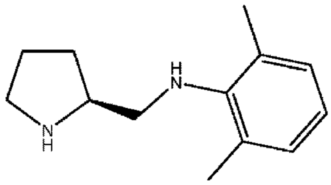 ,

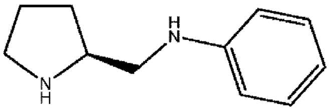 und 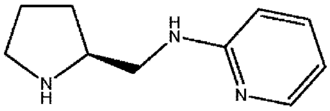

ausgewählt wird.

**5.** Verfahren zur Herstellung einer Verbindung der Formel (V), umfassend die Schritte

i) Verfahren zur Herstellung von Formel (I) nach einem der Ansprüche 1 bis 4;
ii) Reduzieren der Verbindung der Formel (I)

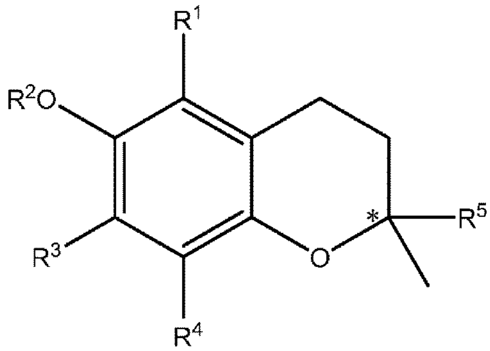

(V).

**6.** Verfahren nach einem der Ansprüche 1 bis 5, **dadurch gekennzeichnet, dass** das Isomer mit der R-Konfiguration an dem durch * gekennzeichneten Chiralitätszentrum in Formel (I) oder (V) gegenüber dem entsprechenden Isomer mit S-Konfiguration an dem Chiralitätszentrum bevorzugt gebildet wird.

**7.** Zusammensetzung, bestehend aus

a) mindestens einer Verbindung der Formel (II-A) und
b) mindestens einem Keton der Formel (II-B) oder einem Ketal davon und
c) mindestens einer chiralen Verbindung der Formel (II-C)

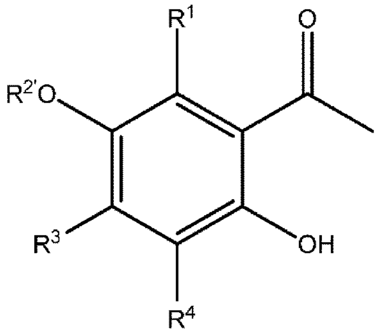

(II-A)

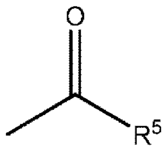

(II-B)

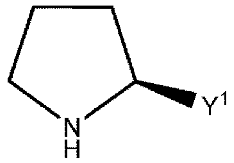

(II-C)

wobei $R^1$, $R^3$ und $R^4$ unabhängig voneinander für Wasserstoff oder Methylgruppen stehen;
$R^{2'}$ für Wasserstoff oder eine Phenol-Schutzgruppe steht;
$R^5$ entweder für eine lineare oder verzweigte vollständig gesättigte $C_{6-25}$-Alkylgruppe oder für eine lineare oder verzweigte $C_{6-25}$-Alkylgruppe mit mindestens einer Kohlenstoff-Kohlenstoff-Doppelbindung steht;
$Y^1$ entweder für $CH_2Y^2$ oder für

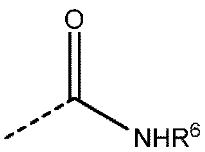

steht, wobei $R^6$ für eine lineare oder verzweigte $C_{1-12}$-Alkylgruppe, die gegebenenfalls ferner mindestens eine aromatische Gruppe und/oder C=0- und/oder NH- und/oder $NH_2$-Gruppe umfasst, steht;
$Y^2$ entweder für OH oder für $OR^7$ oder für $NHR^7$
oder für $NHCOOR^7$ oder für

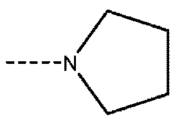

steht, wobei $R^7$ entweder

für eine lineare oder verzweigte $C_{1-12}$-Alkylgruppe, die gegebenenfalls ferner mindestens eine aromatische Gruppe und/oder C=O-und/oder NH- und/oder $NH_2$-Gruppe umfasst,
oder
für eine Arylgruppe oder eine substituierte Arylgruppe oder eine Heteroarylgruppe oder eine substituierte Heteroarylgruppe

steht;

und die gestrichelte Linie bzw. die gestrichelten Linien für die Bindung bzw. die Bindungen, über die der entsprechende Substituent an den Rest der Formel (II-C) gebunden ist, steht bzw. stehen.

8. Zusammensetzung nach Anspruch 7, **dadurch gekennzeichnet, dass** $R^5$ die Formel (III) aufweist:

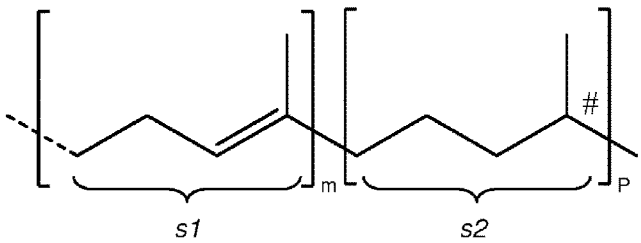

(III)

wobei m und p unabhängig voneinander für einen Wert von 0 bis 5 stehen, mit der Maßgabe, dass die Summe von m und p 1 bis 5 beträgt,
und wobei die durch *s1* und *s2* wiedergegebenen Unterstrukturen in Formel (III) in beliebiger Reihenfolge vorliegen können;
und die gestrichelte Linie für die Bindung steht, über die der Substituent der Formel (III) an den Rest der Verbindung der Formel (II-B) gebunden ist;
und wobei # für ein Chiralitätszentrum steht, offensichtlich mit Ausnahme des Falls, in dem das Zentrum an zwei Methylgruppen gebunden ist.

9. Zusammensetzung nach Anspruch 7 oder 8, **dadurch gekennzeichnet, dass** die Verbindung der Formel (II-C) aus der Gruppe bestehend aus

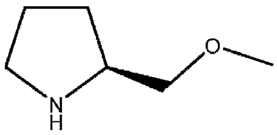 , 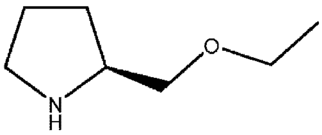 , 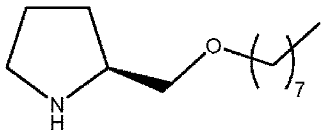 ,

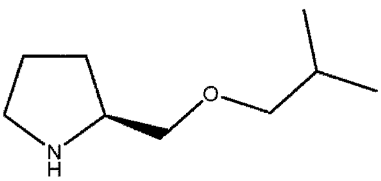 , 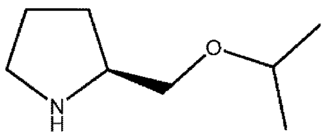 , 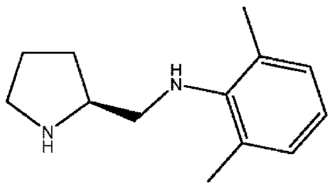 ,

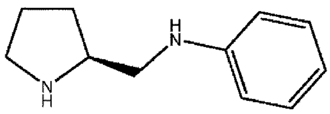 und 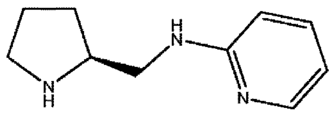

ausgewählt ist.

**Revendications**

1. Procédé de fabrication d'un composé de formule (I)

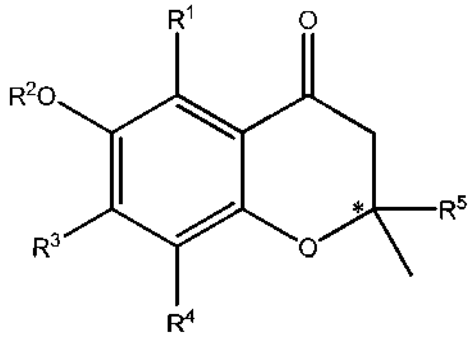

(I)

comprenant l'étape de réaction d'une composition constituée

- d'un composé de formule (II-A) ;
- d'un composé de formule (II-B)
- d'au moins un composé chiral de formule (II-C) ou d'un de ses cétals et

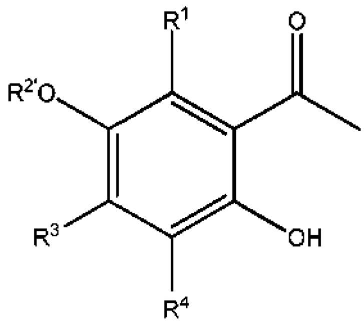

(II-A)

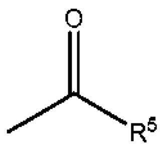

(II-B)

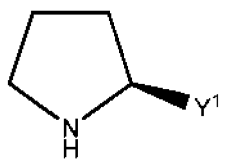

(II-C)

où $R^1$, $R^3$ et $R^4$ sont indépendamment les uns des autres des groupes hydrogène ou méthyle ;
$R^2$ et $R^{2'}$ représentent hydrogène ou un groupe de protection de phénol ;
$R^5$ représente un groupe alkyle en $C_{6-25}$ linéaire ou ramifié, totalement saturé ou un groupe alkyle en $C_{6-25}$ linéaire ou ramifié comprenant au moins une double liaison carbone-carbone ;
$Y^1$ représente $CH_2Y^2$ ou

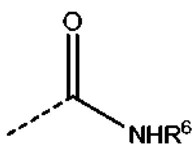

où $R^6$ représente un groupe alkyle en $C_{1-12}$ linéaire ou ramifié qui comprend facultativement en outre au moins un groupe aromatique et/ou un groupe C=O et/ou NH et/ou $NH_2$ ;
$Y^2$ représente OH ou $OR^7$ ou $NHR^7$ ou $NHCOOR^7$ ou

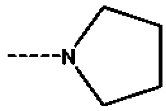

où $R^7$ représente
un groupe alkyle en $C_{1-12}$ linéaire ou ramifié qui comprend facultativement en outre au moins un groupe aromatique et/ou un groupe C=O et/ou NH et/ou $NH_2$
ou
un groupe aryle ou un groupe aryle substitué ou un groupe hétéroaryle ou un groupe hétéroaryle substitué ;
et le (s) trait (s) pointillé (s) représentent la ou les liaison (s) par lesquelles le substituant correspondant est lié au résidu de formule (II-C) ;
et dans lequel * représente le centre chiral de l'isomère chiral de formule (I).

**2.** Procédé selon la revendication 1 **caractérisé en ce que** $R^5$ est de formule (III)

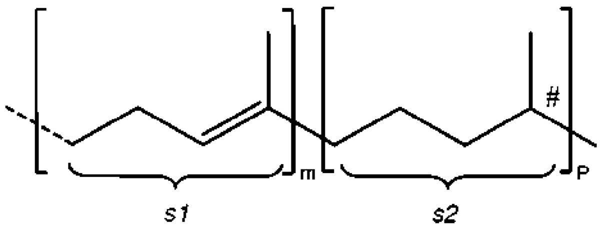

(III)

où m et p désignent indépendamment l'un de l'autre une valeur de 0 à 5 à condition que la somme de m et p soit 1 à 5,
et où les sous-structures dans la formule (III) représentées par *s1* et *s2* puissent être dans une séquence quelconque ;
et le trait pointillé représente la liaison par laquelle le substituant de formule (III) est lié au résidu du composé de formule (II-B) ou de formule (I) ;
et où # représente un centre chiral, évidemment sauf si ledit centre est lié à deux groupes méthyle.

**3.** Procédé selon l'une quelconque des revendications précédentes **caractérisé en ce que**

$R^1 = R^3 = R^4 = CH_3$

ou

$R^1 = R^4 = CH_3$, $R^3 = H$

ou

$R^1 = H$, $R^3 = R^4 = CH_3$

ou

$R^1 = R^3 = H$, $R^4 = CH_3$.

**4.** Procédé selon l'une quelconque des revendications précédentes **caractérisé en ce que** le composé de formule (II-C) est choisi dans le groupe constitué de

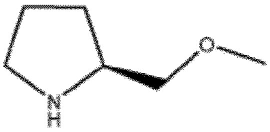
,
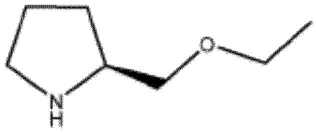
,
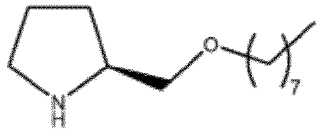
,

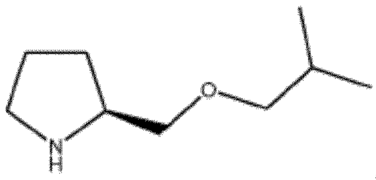, 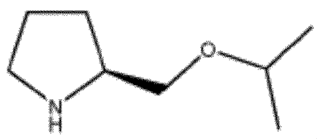, 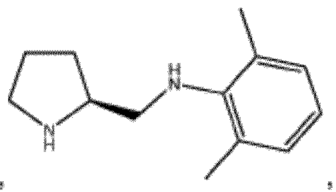,

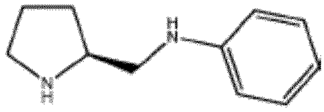 et 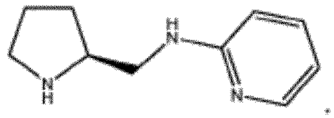.

5. Procédé de fabrication d'un composé de formule (V) comprenant les étapes suivantes

i) procédé de fabrication de formule (I) selon l'une quelconque des revendications 1 à 4 ;
ii) réduction du composé de formule (I)

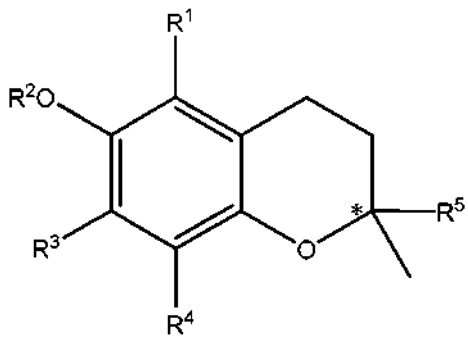 (V).

6. Procédé selon l'une quelconque des revendications 1 à 5, **caractérisé en ce que** l'isomère ayant la configuration R au centre chiral marqué par * dans la formule (I) ou (V) est préferentiellement formé par rapport à l'isomère correspondant ayant la configuration S audit centre chiral.

7. Composition constituée

a) d'au moins un composé de formule (II-A) et
b) d'au moins une cétone de formule (II-B) ou d'un de ses cétals et
c) d'au moins un composé chiral de formule (II-C)

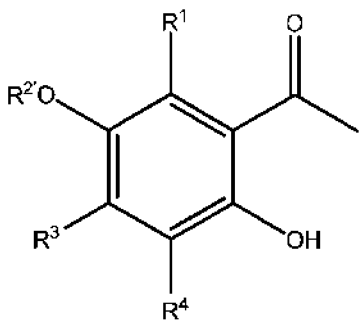 (II-A)

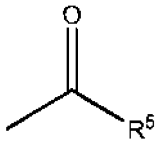

(II-B)

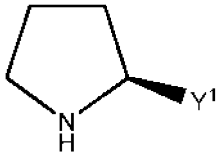

(II-C)

où $R^1$, $R^3$ et $R^4$ sont indépendamment les uns des autres des groupes hydrogène ou méthyle ;
$R^{2'}$ représente hydrogène ou un groupe de protection de phénol ;
$R^5$ représente un groupe alkyle en $C_{6-25}$ linéaire ou ramifié totalement saturé ou un groupe alkyle en $C_{6-25}$ linéaire ou ramifié comprenant au moins une double liaison carbone-carbone ;
$Y^1$ représente $CH_2Y^2$ ou

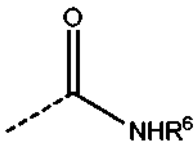

où $R^6$ représente un groupe alkyle en $C_{1-12}$ linéaire ou ramifié qui comprend facultativement en outre au moins un groupe aromatique et/ou un groupe C=O et/ou NH et/ou $NH_2$ ;
$Y^2$ représente OH ou $OR^7$ ou $NHR^7$ ou $NHCOOR^7$ ou

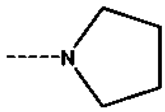

où $R^7$ représente
un groupe alkyle en $C_{1-12}$ linéaire ou ramifié qui comprend facultativement en outre au moins un groupe aromatique et/ou un groupe C=O et/ou NH et/ou $NH_2$
ou
un groupe aryle ou un groupe aryle substitué ou un groupe hétéroaryle ou un groupe hétéroaryle substitué ;
et le (s) trait (s) pointillé (s) représentent la ou les liaison(s) par lesquelles le substituant correspondant est lié au résidu de formule (II-C).

8. Composition selon la revendication 7, **caractérisée en ce que** $R^5$ est de formule (III)

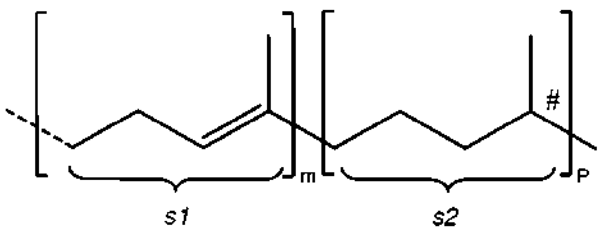

(III)

où m et p désignent indépendamment l'un de l'autre une valeur de 0 à 5 à condition que la somme de m et p

soit 1 à 5,

et où les sous-structures dans la formule (III) représentées par *s1* et *s2* puissent être dans une séquence quelconque ;

et le trait pointillé représente la liaison par laquelle le substituant de formule (III) est lié au résidu du composé de formule (II-B);

et où # représente un centre chiral, évidemment sauf si ledit centre est lié à deux groupes méthyle.

9. Composition selon la revendication 7 ou 8 **caractérisée en ce que** le composé de formule (II-C) est choisi dans le groupe constitué de

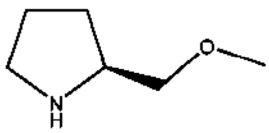,
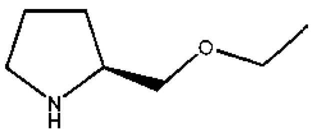,
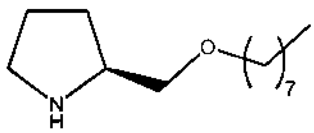,
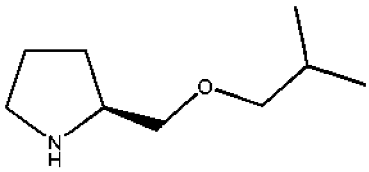,
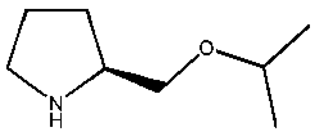,
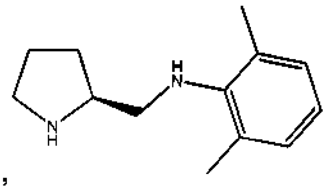,
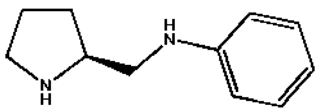; et
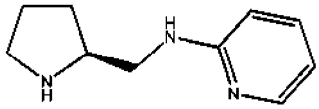.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009019132 A
- WO 2006066863 A1
- WO 2012152779 A1
- US 6096907 A
- EP 0989126 A
- EP 0989126 A1, Baldenius


### Non-patent literature cited in the description


- **H. WEISER et al.** *J. Nutr.,* 1996, vol. 126 (10), 2539-49
- **KABBE ; HEITZER.** *Synthesis,* 1978, 888-889
- **G. MANECKE ; G. BOURWIEG.** *Chem. Ber.,* 1962, vol. 95, 1413-1416
- **THOMAS ELTZ et al.** *J. Chem. Ecol.,* 2010, vol. 36, 1322-1326
- **PÉRIO et al.** *Tetrahedron Letters,* 1997, vol. 38 (45), 7867-7870
- **LORETTE ; HOWARD.** *J. Org. Chem.,* 1960, 521-525
- **P. SCHUDEL et al.** *Helv. Chim. Acta,* 1963, vol. 46, 2517-2526